# EUROPEAN PATENT SPECIFICATION

(11) **EP 1 757 231 B1**
(45) Date of publication and mention of the grant of the patent: **08.04.2009**
(21) Application number: 05018376.3
(22) Date of filing: 24.08.2005
(51) Int. Cl.: A61B 17/00

(54) **Tissue separator particularly for removing the saphenous vein**
Gewebetrenner insbesondere zum Entfernen der Saphenusvene
Séparateur de tissus notamment pour retirer la veine saphene

(43) Date of publication of application: 28.02.2007
(73) Proprietor: Terrini, Alberto, 30174 Venezia (IT)
(72) Inventor: Terrini, Alberto, 30174 Venezia (IT)
(74) Representative: Alagem Modiano, Lara S.

(56) References cited:
- US-A- 5 928 138
- US-A- 6 080 102
- US-B1- 6 228 024

## Description

The invention relates to a tissue separator particularly for removing the saphenous vein.

It is known that removal of the saphenous vein from the leg of a patient is currently performed, according to a widely practiced technique, by manually inserting a separator, which keeps the tissues separated from the vein and forms an operating tunnel, within which suitable instruments are inserted for cutting the collateral veins connected to the saphenous vein, so as to free said vein and allow its extraction by sliding.

The entire operation, which can be preceded by a preoperative step for separating the tissues in contact with the vein, performed by means of an instrument that is similar to the separator mentioned above, is managed by the surgeon by means of an endoscope, which allows to view the situation at the operating tunnel.

The devices for separating tissues from the saphenous vein of the background art have various shapes, all of which, however, are not free from the disadvantageous characteristic of not providing the maximum operating freedom for the instruments designed to cut the collateral veins, particularly due to the bulk caused by the duct that is designed to accommodate the endoscope.

A tissue separator according to the preamble of claim 1 is known from U.S. Patent n. 5,928,138.

U.S. Patent n. 6,080,102 discloses a tissue separator comprising a body formed into an elongated tube into which the hard endoscope can be inserted; a large-diameter pipe formed in the body at a position adjacent to the operator's hand; and a transparent leading end connected to the leading end of the body and arranged to cover the leading end section of the hard endoscope.

The aim of the present invention is to provide a separator that allows surgeons to remove the saphenous vein in optimum conditions.

The proposed aim is achieved by the subject-matter of claim 1.

Further characteristics and advantages of the present invention will become better apparent from the following detailed description of a preferred but not exclusive embodiment of the invention, illustrated by way of non-limiting example in the accompanying drawing, wherein:

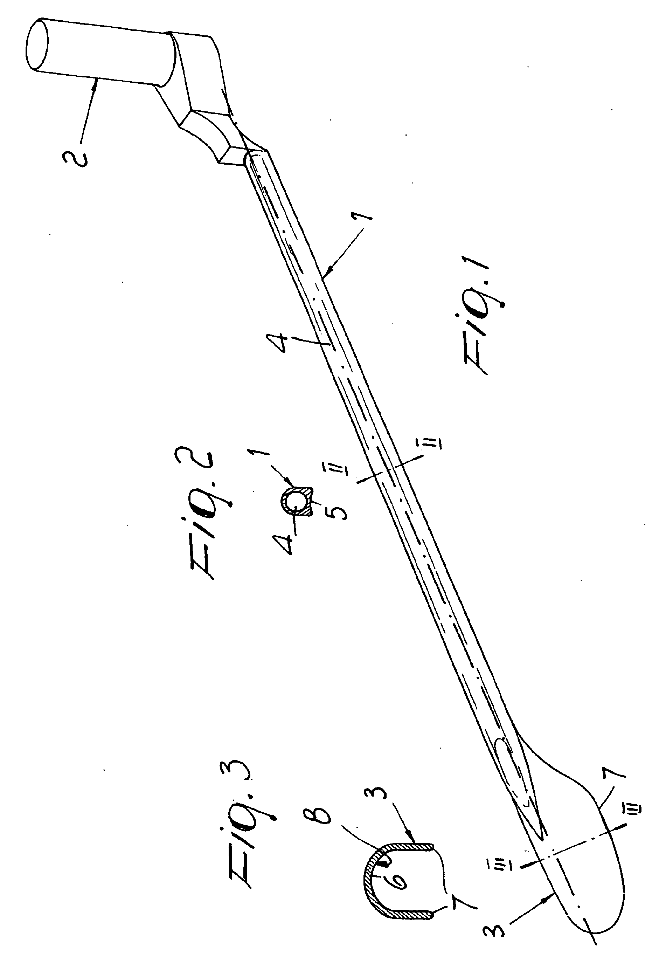

Figure 1 is a perspective view of the invention;
Figures 2 and 3 are sectional views, taken respectively along the lines II-II and III-III of Figure 1.

With reference to the figures, it can be seen that the separator according to the invention comprises the rod 1, which is provided at its ends respectively with the grip handle 2 and with the bowl 3.

The rod 1 comprises internally the longitudinal cavity 4, which is designed to accommodate the endoscope that allows the operator to see what is happening in the portion of space that corresponds to the bowl 3, and forms the continuous and slightly arched or slightly arc-like surface 5 in the region that is designed to be directed toward the saphenous vein when the separator is made to penetrate gradually the leg of the patient; this configuration ensures particularly easy conditions in the passage of the instruments that must reach the space at the bowl 3.

The inside curve 6 of said bowl 3 is shaped so as to form a continuous arc-like surface 8, which is completely devoid of any protruding parts whose bulk might restrict the operating freedom of the instruments designed to cut the collateral veins: essentially, a tunnel that provides optimum functional conditions for the instruments accommodated thereat is formed below the bowl 3.

Finally, it should be noted that the bowl 3 has longitudinal edges 7 which are slightly inclined with respect to the longitudinal axis of the rod 1 in order to facilitate the action of the operator in the step for inserting the separator in the leg of the patient.

The described invention is susceptible of numerous modifications and variations, all of which are within the scope of the inventive concept as defined by the appended claims; thus, for example, it is possible to provide a U-shaped element suitable to support the rod 1 by resting against the leg of the patient and to allow the passage of the instruments designed to cut the collateral veins.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A tissue separator, particularly for removing the saphenous vein, comprising a rod (1) which comprises internally a longitudinal cavity (4), which is suitable to accommodate an endoscope, said rod (1) being provided with a grip handle (2) at one end and, at the other end, with a bowl (3) in which the inside curve (6) forms a continuous arc-like surface (8) suitable to form an operating tunnel, **characterized in that** the rod (1) forms on its outside a continuous and slightly arc-like surface (5) at the region that faces the saphenous vein and the longitudinal edges (7) of the surface (8) of the bowl (3) have a slight inclination with respect to the longitudinal axis of the rod (1).

2. The device according to claim 1, **characterized in that** it further comprises a U-shaped element suitable to support the rod (1) by resting against the leg of a patient so as to allow passage of additional instruments.

## Patentansprüche

1. Gewebetrenneinrichtung, insbesondere zum Entfernen der Vena Saphena, mit einem Stab (1), der innen einen longitudinalen Hohlraum (4) aufweist, der ein Endoskop aufnehmen kann, wobei der Stab (1) an einem Ende mit einem Handgriff (2) versehen ist und am anderen Ende mit einem Becher (3) versehen ist, in dem die Innenkrümmung (6) eine ununterbrochene bogenähnliche Oberfläche (8) bildet, die einen Operationstunnel bilden kann, **dadurch gekennzeichnet, dass** der Stab (1) an seiner Außenseite in dem Vena Saphena zugewandten Bereich eine ununterbrochene und leicht bogenförmige Oberfläche (5) bildet und die Längskanten (7) der Oberfläche (8) des Bechers (3) eine leichte Neigung in Bezug auf die Längsachse des Stabs (1) haben.

2. Vorrichtung nach Anspruch 1, **dadurch gekennzeichnet, dass** sie ferner ein U-förmiges Element umfasst, das den Stab (1) unterstützen kann, indem es auf dem Bein eines Patienten aufliegt, um den Durchgang zusätzlicher Instrumente zuzulassen.

## Revendications

1. Séparateur de tissus, en particulier pour retirer la veine saphène, comprenant une tige (1) qui comprend à l'intérieur une cavité longitudinale (4), qui est appropriée pour recevoir un endoscope, ladite tige (1) étant munie d'une poignée de saisie (2) à une extrémité, et, à l'autre extrémité, d'un bol (3) dans lequel la courbe intérieure (6) forme une surface en forme d'arc continue (8) appropriée pour former un tunnel opérationnel, **caractérisé en ce que** la tige (1) forme sur son extérieur une surface continue et légèrement en forme d'arc (5) dans la région qui est dirigée vers la veine saphène et **en ce que** les bords longitudinaux (7) de la surface (8) du bol (3) ont une légère inclinaison par rapport à l'axe longitudinal de la tige (1).

2. Dispositif selon la revendication 1, **caractérisé en ce qu'**il comprend de plus un élément en forme de U approprié pour supporter la tige (1) en reposant contre la jambe d'un patient de façon à permettre le passage d'instruments additionnels.
